# EUROPÄISCHE PATENTSCHRIFT

(11) **EP 2 725 096 B1**
(45) Veröffentlichungstag und Bekanntmachung des Hinweises auf die Patenterteilung: **15.04.2015**
(21) Anmeldenummer: 13187090.9
(22) Anmeldetag: 02.10.2013
(51) Int. Cl.: C12M 1/42, C12M 1/00, C12M 1/33, C12N 1/06

(54) **Zum Lysieren einsetzbare Vorrichtungen, Verfahren und System**
Devices for lysing, method and system
Dispositifs, procédé et système pouvant être utilisés pour la lyse

(30) Priorität: 25.10.2012 DE 102012219575
(43) Veröffentlichungstag der Anmeldung: 30.04.2014
(73) Patentinhaber: Robert Bosch GmbH, 70442 Stuttgart (DE)
(72) Erfinder: Daub, Martina, 71287 Weissach (DE); Rupp, Jochen, 70178 Stuttgart (DE); Rothacher, Peter, 76646 Bruchsal (DE)

(56) Entgegenhaltungen:
- EP-A1- 1 359 415
- EP-A1- 2 065 085
- WO-A1-99/28742
- CN-U- 202 576 411
- JP-A- 406 305 703
- US-A1- 2007 231 346
- US-B1- 6 686 195

## Beschreibung

Die vorliegende Erfindung bezieht sich auf Vorrichtungen, ein Verfahren und System, die zum Lysieren mittels Ultraschalls einsetzbar sind, bzw. eingesetzt werden.

### Stand der Technik

Das Lysieren als ein Verfahren zum Auflösen von Zellmembranen und somit zum Bewirken des Zerfalls von Zellen ist allgemein bekannt. Zum Lysieren können verschiedene Verfahren eingesetzt werden, darunter auch das Lysieren mittels Übertragung/Anwendung der Energie auf die Probe oder das Lysat, was allgemein bekannt ist. Im Nachfolgenden wird die vorliegende Erfindung hinsichtlich der Übertragung mittels Ultraschalls erläutert. Dabei ist aber anzumerken, dass das Verwenden des Ultraschalls als eine Art der Energie nur beispielhaft ist und dass die vorliegende Erfindung nicht auf diese beschränkt ist. D.h. es können auch anderweitige geeignete Energieübertragungen eingesetzt werden, beispielsweise Magnetaktoren.

Durch Anwenden des Ultraschalls auf eine (Proben- oder Lyse-) Flüssigkeit mit den aufzulösenden Zellen werden die Zellen durch Scherkräfte, die auf die Zellmembranen und/oder Zellwände wirken, zerstört. Üblicher Weise wird ein Energie- (Ultraschall-) -übertragendes Element in die Probe eingetaucht und mit Ultraschall beschallt. Das Einkoppeln der Energie in die Probe durch das energieübertragende Element führt zur Kavitation und/oder zum Erzeugen von Scherkräften in der Probe, die dann zum Zerstören oder Zerfall der Zellen bzw. deren widertandsfähigen Zellmembranen in der Probe führen.

Die bekannten Verfahren zum Lysieren mittels Ultraschalls haben jedoch eine Vielzahl an Nachteilen. Beim Eintauchen des energieübertragenden Elements können Verunreinigungen in die Probe eingetragen werden. Dieses ist insbesondere dann der Fall, wenn das energieübertragende Element mehrfach verwendet wird und nach jedem Lysiervorgang gereinigt werden muss. Zum einen kann eine Probe mit Bestandteilen einer vorherigen Probe kontaminiert werden. Ferner ist eine Verunreinigung durch Reinigungsmittel nicht ausgeschlossen. Ferner entsteht bei jedem weiteren Lysieren ein hoher manueller Aufwand zum Vorbereiten des Lysiervorgangs. Der Aufwand sowie der Zeitbedarf beschränken den potenziellen Durchsatz an Proben.

Einige bekannte Verfahren versuchen, die oben genannten Nachteile dadurch zu beheben, dass das Lysieren in einem Ultraschallbad erfolgt. Hierzu wird die zu lysierende Probe in einen Behälter aufgenommen, welcher in eine beschallte Flüssigkeit eines Bads getaucht wird. Diese beschallte Flüssigkeit wird auch Ultraschallbad genannt. Im Ultraschallbad wird die dem Lysieren zu unterziehende Probe in der Regel für eine bestimmte Zeit einem konstanten Ultraschallfeld ausgesetzt. Allerdings ist die Energieübertragung durch das Ultraschallbad erheblich schwächer. Ein weiterer Nachteil ist die Pflege der Flüssigkeit des Ultraschallbads, da diese eine Verkeimung und/oder Veralgung erfahren kann. Ferner bleibt die Flüssigkeit des Ultraschallbads am Behälter mit der lysierten Probe haften und kann zu Verunreinigungen aus der Ultraschallbadflüssigkeit führen und/oder störend auf die Automatisierbarkeit des gesamten Prozesses wirken. Ferner kann das Lysieren mittels Ultraschallbads Mängel bezüglich einer genauen Reproduzierbarkeit mit sich bringen, da die Reproduzierbarkeit stark von der Position des Behälters mit der zu lysierenden Probe im Ultraschallbad abhängt, wobei zusätzlich auch die Anordnung der Wandler um das Ultraschallbecken bei einem wiederholten Lysieren genau eingehalten werden muss. Ferner ist die Verteilung des Ultraschalls im Ultraschallbad nicht homogen. Dadurch kann der Energieeintrag nicht optimal dosiert werden, um die Zellen zu lysieren (bestimmt durch minimale Energie) und die beim Lysieren freigesetzten Bestandteile intakt zu halten (bestimmt durch maximale Energie).

Somit besteht nach wie vor Bedarf nach Lyse-Verfahren und -Vorrichtungen, durch welche die oben genannten Nachteile behoben werden können.

Aus dem Dokument JP 406305703 A ist ein Fotobioreaktor bekannt, der mittig eine zum Einführen einer lichtemmitierenden Quelle transparente Röhre aufweist.

### Offenbarung der Erfindung

Die Erfindung geht aus von Vorrichtungen, einem Verfahren und einem System nach Gattung der unabhängigen Ansprüche. Weitere Ausgestaltungen der vorliegenden Erfindung können den abhängigen Ansprüchen entnommen werden.

Die Idee der vorliegenden Erfindung besteht darin, das Eintauchen des energieübertragenden Elements (z.B. eines Resonators, einer Sonotrode oder eines Horns, die als Ultraschall übertragende allgemein bekannt sind) in eine dem Lysieren zu unterziehende Probe oder das Kontaktieren der dem Lysieren zu unterziehenden Probe oder Probe durch das energieübertragende Element derart zu ermöglichen, dass die oben genannten Nachteile nicht entstehen. Dafür wird ein aufnehmendes Element eingesetzt, das sich vor dem Eintauchen in die Probe zumindest teilweise um und/oder an das energieübertragende Element legt. Das aufnehmende Element umhüllt zumindest einen Teil des energieübertragenden Elements derart und/oder das aufnehmende Element liegt an zumindest einen Teil des energieübertragenden Elements derart an, dass das aufnehmende Element mit dem aufgenommenen Teil des energieübertragenden Elements im direkten Kontakt ist. Das direkte Umhüllen und/oder das direkte Anliegen kann derart sein, dass keine Probeflüssigkeit, keine weiteren Flüssigkeiten und/oder Zwischenräume zwischen dem aufnehmenden Element und dem energieübertragenden Element gegeben sind. Ferner können das aufnehmende Element und der aufgenommene Teil des energiebertragenden Elements (z.B. eine Sonotrode oder ein Horn) einen (im Wesentlichen) spaltfreien Kontakt aufweisen. Der umhüllte Teil des energieübertragenden Elements und/oder der Teil des energieübertragenden Elements, an den das aufnehmende Element anliegt, entspricht zumindest demjenigen Teil, der in die Probe eingetaucht wird. Der umhüllte Teil kann auch das gesamte energieübertragende Element (wie z.B. Sonotrode oder Horn) umfassen. Die Umhüllung kann zeitlich begrenzt sein. D.h. das energieübertragende Element wird für eine bestimmte Zeit, insbesondere für die Zeit, in der der Lysiervorgang vorgenommen wird, mit dem aufnehmenden Element umhüllt. Das aufnehmende Element kann auch zeitlich begrenzt dem aufgenommenen Teil des energieübertragenden Elements anliegen. D.h. das aufnehmende Element liegt für eine bestimmte Zeit, insbesondere für die Zeit, in der der Lysiervorgang vorgenommen wird, dem energieübertragenden Element an.

Die vorliegende Erfindung ermöglicht ein verbessertes Lysieren. So zum Beispiel ermöglicht die vorliegende Erfindung ein kontaminationsfreies Lysieren von Proben, da das (zumindest teilweise) in das aufnehmende Element aufgenommene energieübertragende Element ohne einen direkten Kontakt zu der dem Lysieren zu unterziehenden Probe in diese Probe eingetaucht werden kann oder mit dieser Probe in Verbindung treten kann. Ferner kann das Lysieren mit einem geringen manuellen Aufwand vollzogen werden, wodurch auch das Automatisieren des Lysierprozesses ermöglicht wird. Vor allem erlaubt die vorliegende Erfindung eine gute Reproduzierbarkeit eines jeden Lysiervorgangs. Ferner wird eine effektive und optimale Dosierung des Energieeintrags von dem energieübertragenden Element über das aufnehmende Element sichergestellt. Es ist somit möglich, die Zellen aufzuschließen bzw. zu lysieren und gleichzeitig die Bestandteile der Zellen intakt zu halten. Die vorliegende Erfindung bietet somit eine optimale Energieeinkopplung in die zu lysierende Probe, die gut und effektiv zu steuern ist. Die Dauer des Lysiervorgangs und/oder die Vorbereitung des Lysiervorgangs wird deutlich reduziert.

In nachfolgenden Figuren wird die vorliegende Erfindung konkreter unter Bezugnahme auf beispielhafte Ausführungsformen der vorliegenden Erfindung beschrieben. Zu Klarheitszwecken werden in den Figuren gleiche oder ähnliche Elemente mit den gleichen oder ähnlichen Bezugszeichen bezeichnet.
Fig. 1 zeigt eine zum Verwenden beim Lysieren ausgestaltete Vorrichtung gemäß einer Ausführungsform der vorliegenden Erfindung;
Fig. 2 zeigt das Einführen des energieübertragenden Elements in das aufnehmende Element gemäß einer Ausführungsform der vorliegenden Erfindung;
Fig. 3 zeigt eine zum Verwenden beim Lysieren ausgestaltete Vorrichtung gemäß einer Ausführungsform der vorliegenden Erfindung;
Fig. 4 zeigt die Vorrichtung gemäß der Ausführungsform, wenn das aufnehmende Element das energieübertragende Element zumindest teilweise zum Lysieren einer Probe aufgenommen hat;
Fig. 5 zeigt eine zum Verwenden beim Lysieren ausgestaltete Vorrichtung gemäß einer Ausführungsform der vorliegenden Erfindung;
Fig. 6 zeigt eine zum Verwenden beim Lysieren ausgestaltete Vorrichtung gemäß einer Ausführungsform der vorliegenden Erfindung;
Fig. 7a zeigt das spaltbehaftete Bilden eines Kontakts zum energiegebenden Element gemäß bekannter Verfahren zum Lysieren mit energiegebenden Elementen;
Fig. 7b zeigt das spaltlose Bilden eines Kontakts zum energiegebenden Element gemäß einer Ausführungsform der vorliegenden Erfindung;
Fig. 8 zeigt eine zum Verwenden beim Lysieren ausgestaltete Vorrichtung gemäß einer Ausführungsform der vorliegenden Erfindung;
Fig. 9 zeigt gemäß einer Ausführungsform der vorliegenden Erfindung die Schritte eines Verfahrens zum Einführen eines energieübertragenden Elements in ein aufnehmendes Element zum Lysieren einer Probe; und
Fig. 10 zeigt gemäß einer Ausführungsform eine Vorrichtung, die ausgestaltet ist, das vorstehend erwähnte Verfahren auszuführen.

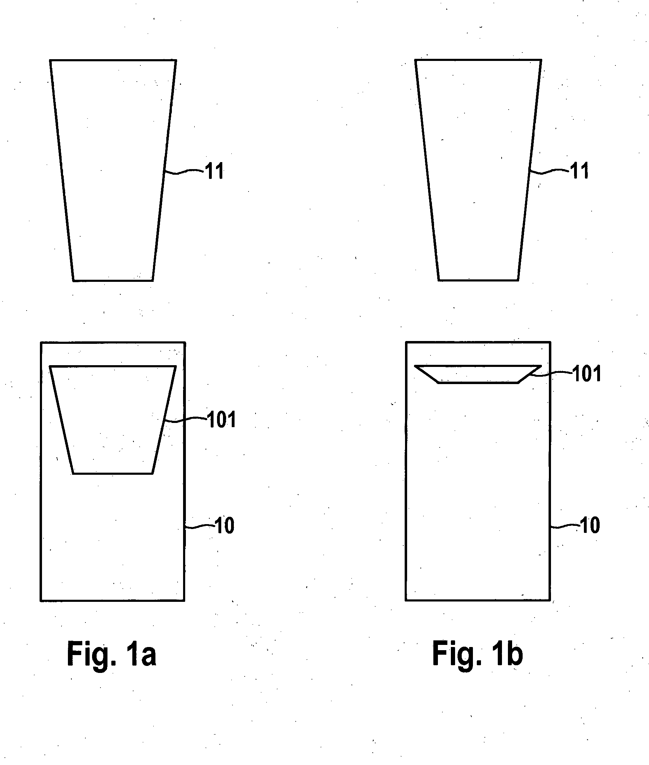

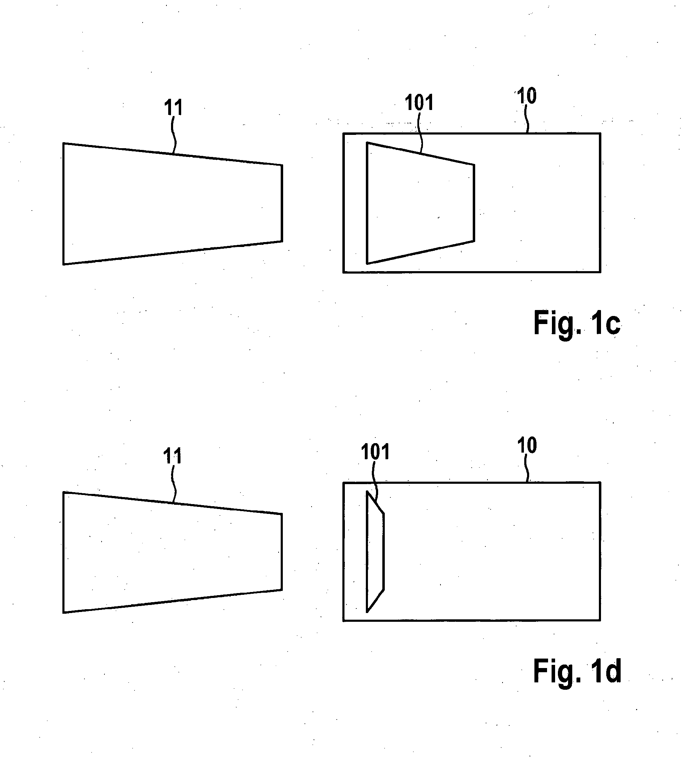

Fig. 1 zeigt eine Vorrichtung 10 gemäß einer (allgemeinen) Ausführungsform der vorliegenden Erfindung, wobei die Vorrichtung zum Verwenden beim Lysieren einer dem Lysieren zu unterziehenden Probe (z.B. mittels Ultraschalls) ausgestaltet ist. Das Lysieren kann mittels Ultraschalls erfolgen. Die Vorrichtung 10 weist ein aufnehmendes Element 101 auf, das ausgestaltet ist: ein energieübertragendes Element 11 zumindest teilweise derart aufzunehmen, dass das aufnehmende Element 101 sich um den aufgenommenen Teil des energieübertragenden Elements 11 legt und den aufgenommenen Teil des energieübertragenden Elements 11 umhüllt und/oder dass sich das aufnehmende Element 101 an den aufgenommenen Teil des energieübertragenden Elements 11 legt; und mit dem energieübertragenden Element 11, dessen aufgenommene Teil durch das aufnehmende Element 101 umhüllt ist und/oder an dessen aufgenommenen Teil das aufnehmende Element anliegt, das Lysieren zu bewirken, wenn das aufnehmende Element 101, das den aufgenommenen Teil des energieübertragenden Elements 11 umhüllt und/oder das an den den aufgenommenen Teil des energieübertragenden Elements 11 anliegt, in eine dem Lysieren zu unterziehende Probe eingetaucht ist oder mit der dem Lysieren zu unterziehenden Probe in Kontakt steht. Wie bereits oben erwähnt, ermöglicht die Vorrichtung 10 ein kontanimationsfreies Lysieren von Proben, da das (zumindest teilweise) in das aufnehmende Element 101 aufgenommene energieübertragende Element 11 ohne einen direkten Kontakt zu der dem Lysieren zu unterziehenden Probe in diese Probe eingetaucht werden kann oder mit dieser Flüssigkeit in Verbindung treten kann. Ferner wird zum Lysieren und zwischen den Lysiervorgängen verschiedener nacheinanderfolgender Proben ein geringer manueller Aufwand benötigt. Somit wird durch die Vorrichtung 10 auch das Automatisieren und Hintereinanderschalten mehrerer Lysierprozesse ermöglicht. Darüber hinaus erlaubt die Vorrichtung 10 eine gute Reproduzierbarkeit eines jeden Lysiervorgangs. Ferner wird eine effektive und optimale Dosierung des Energieeintrags von dem energieübertragenden Element 11 über das aufnehmende Element 101 ermöglicht. Es ist somit möglich, die Zellen aufzuschließen bzw. zu lysieren und gleichzeitig die Bestandteile der Zellen intakt zu halten. Die Vorrichtung 10 führt zu beliebigen Kombinationen der oben genannten positiven Wirkungen. Das energieübertragende Element 11 kann beispielsweise ausgestaltet sein Ultraschall zu übertragen. Dabei kann das energieübertragende Element 11 beispielsweise ein Resonator, eine Sonotrode oder ein Horn sein. Die vorliegende Erfindung erlaubt verschiedene geeignete Ausgestaltungen des energieübertragenden Elements 11 und verschiedene geeignete Arten und Weisen des Übertragens der Energie.

Das energieübertragende Element 11 und das aufnehmende Element 101 gehen gemäß der vorliegenden Ausführungsform eine vorübergehende Verbindung ein, d.h. das Umhüllen des aufgenommenen Teils des energieübertragenden Elements 11 durch das aufnehmende Element 101 oder das Anliegen des aufnehmenden Elements 101 an den aufgenommenen Teil des energieübertragenden Elements 11 ist zeitlich begrenzt. Nach dem Lysiervorgang wird das energieübertragende Element 11 oder der entsprechende aufgenommene Teil des energieübertragenden Elements 11 aus dem aufnehmenden Element hinausgeführt. Der Kontakt zwischen dem energieübertragenden Element 11 und dem aufnehmenden Element 101 wird nach dem Abschluss des Lysiervorgangs aufgelöst (z.B. durch Hinausziehen des energieübertragenden Elements 11 aus dem aufnehmenden Element 101 und/oder durch Lösen des (direkten) Kontakts zwischen dem energieübertragenden Element 11 und dem aufnehmenden Element 101).

Gemäß dem vorliegenden Ausführungsbeispiel ist das aufnehmende Element 101 ausgestaltet, sich direkt um und/oder an den aufgenommenen Teil des energieübertragenden Elements 11 zu legen und in direktem Kontakt zu dem aufgenommenen Teil des energieübertragenden Elements 11 zu sein. Direkter Kontakt bedeutet, dass das energieübertragende Element 11 und das aufnehmende Element 101 sich direkt (und im Wesentlichen oder gänzlich spaltfrei) berühren. D.h. durch den direkten Kontakt berühren sich das aufnehmende Element 101 und das energieübertragende Element 11 derart, dass sich keine Proben, Flüssigkeiten und/oder Stoffe zwischen den beiden Elementen 101, 11 befinden.

Gemäß einer Ausführungsform, die mit allen der erläuterten Ausführungsformen kombinierbar ist, ist das aufnehmende Element 101 als ein elastisches und derofmierbares Element ausgestaltet. Gemäß der vorliegenden Ausführungsform ist das aufnehmende Element 101 als ein elastisches und deformierbares Element und/oder als eine Membran ausgestaltet. Dadurch wird ein gutes Aufnehmen und Kontaktieren des energieübertragenden Elements 11 durch das aufnehmende Element 101 ermöglicht, wodurch die oben bezüglich der Vorrichtung 10 genannten positiven Eigenschaften verstärkt werden. Das aufnehmende Element 101 kann zumindest teilweise elastisch und deformierbar ausgestaltet sein. D.h. es kann gänzlich elastisch und deformierbar sein oder zumindest ein bestimmter Teil des aufnehmenden Elements 101 kann elastisch und deformierbar sein. Dadurch kann das aufnehmende Element 101 flexibel ausgestaltet werden. Dabei kann eine bessere Anpassungsfähigkeit an das energieübertragende Element 11 erreicht werden. Ferner kann auch die Funktion des direkten Überzugs und/oder des direkten Anliegens besser unterstützt werden. Z.B. kann es für eine bessere Passform an das energieübertragende Element 11 sinnvoll sein, dass das aufnehmende Element 101 an zumindest einem Teil fester und ggf. nicht oder weniger elastisch oder nicht oder weniger deformierbar ist, während an zumindest einem weiteren Teil die Eigenschaften der Elastizität und Deformierbarkeit gegeben sind. Je besser das aufnehmende Element 101 sich an das energieübertragende Element 11 anpasst, desto besser wird der direkte Kontakt zwischen beiden Elementen 101, 11 ausgebildet, was wiederum einen jeden der in der Anmeldung genannten weiteren Vorteile verstärkt (z.B. eine bessere Energieeinkopplung kann gewährleistet werden usw.). Das energieübertragende Element 11 ist gemäß der vorliegenden Ausführungsform eine Sonotrode oder ein Horn. Dadurch wird eine gute Energieeinkopplung und Beschallung in der Probeflüssigkeit ermöglich, was ebenfalls die oben genannten positiven Eigenschaften unterstützt. Wie bereits bekannt, erlauben die Sonotrode und das Horn das Beschallen der Probeflüssigkeit mittels Ultraschalls, da sowohl die Sonotrode als auch das Horn Ultraschall übertragende Elemente sind.

Bei der Erläuterung der nachfolgenden Ausführungsformen wird weiterhin der Begriff "aufnehmendes Element" verwendet. Es ist aber anzumerken, dass die vorliegende Erfindung unter dem "aufnehmenden Element" generell auch ein "elastisches und deformierbares Element" und/oder "Membran" versteht und dass die vorliegende Anmeldung bei jeder Spezifizierung des "aufnehmenden Elements" auch das "elastische und deformierbare Element" und die "Membran" .spezifiziert.

Ebenso wird beim Erläutern der vorliegenden Erfindung der Begriff eines "energieübertragenden Elements" verwendet. Dabei ist anzumerken, dass die vorliegende Erfindung unter dem "energieübertragenden Element" generell auch eine Sonotrode oder ein Horn versteht. Dabei wird durch die Sonotrode oder das Horn Ultraschall als Energie übertragen. Anzumerken ist auch, dass auch weitere energieübertragende Elemente durch die vorliegende Erfindung angewendet werden können. Daher spezifiziert die vorliegende Anmeldung bei jeder Spezifizierung des energieübertragenden Elements auch eine Sonotrode, ein Horn oder ein anderweitiges geeignetes energieübertragendes Element.

Fig. 1 setzt sich aus vier Teilfiguren 1a bis 1d zusammen. Gemäß den Ausführungsformen der Figuren 1a und 1c ist das aufnehmende Element 101 bezüglich der Form des energieübertragenden Elements 11 vorgeformt. Dadurch wird ein gutes Aufnehmen des energieübertragenden Elements 11 ermöglicht, wodurch die oben bezüglich der Vorrichtung 10 genannten positiven Eigenschaften verstärkt werden. Das Aufnehmen des energieübertragenden Elements 11 umfasst dabei das Umhüllen des energieübertragenden Elements 11 durch das aufnehmende Element 101 und/oder das Anliegen des aufnehmenden Elements 101 an das energieübertragende Element. Insbesondere kann ein direkterer Kontakt zwischen dem aufnehmenden Element 101 und dem energieübertragenden Element 101 ermöglicht werden. Gemäß den Ausführungsformen der Figuren 1b und 1d ist das aufnehmende Element 101 nicht vorgeformt. Durch die Eigenschaft der Elastizität können jedoch auch hier die positiven Eigenschaften der Figuren 1a und 1c erreicht werden.

Das aufnehmende Element 101 (d.h. auch das elastische und deformierbare Element oder Membran 101) kann aus einem Polymer oder aus Elastomer (z.B. Gummi) bestehen. Polymer kann beispielsweise PC, COP, COC, PP, PE, PMMA, PET, PEN, Silicon oder TPE sein. Diese Stoffe bieten gute Eigenschaften der Elastizität und der Deformierbarkeit, was die oben genannten positiven Wirkungen bestärkt.

Dabei kann das aufnehmende Element 101 typischerweise die folgende Dicke aufweisen: 10 □m bis 400 □m; 50 □m bis 1 mm; 300 □m bis 400 µm; oder 375 µm. Insgesamt kann die Dicke von 10 □m bis 1mm sein. Durch diese Dicken werden die Eigenschaften der Elastizität und der Deformierbarkeit des aufnehmenden Elements 101 unterstützt, was ebenfalls die oben genannten positiven Wirkungen bestärkt.

Wie die Teilfiguren 1a bis 1d zeigen, kann das energieübertragende Element 11 aus verschiedenen Richtungen und in verschiedenen Winkeln in das aufnehmende Element 101 eingeführt werden. Fig. 1a und Fig. 1b zeigen dabei beispielhaft das Einführen von oben und Fig. 1c und Fig. 1d zeigen beispielhaft das Einführen von der Seite.

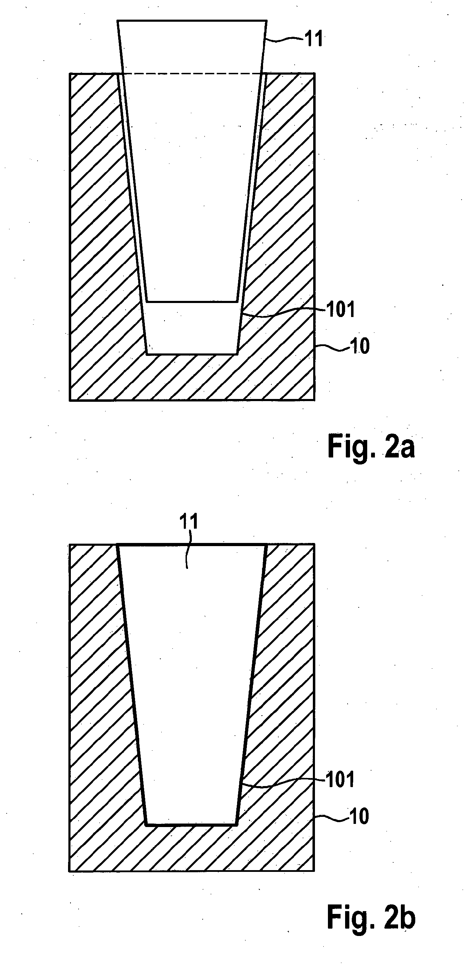

Fig. 2 zeigt das Einführen des energieübertragenden Elements 11 in das aufnehmende Element 101 oder gemäß einer Ausführungsform der vorliegenden Erfindung. Gemäß der vorliegenden Ausführungsform ist das aufnehmende Element 101 vorgeformt. Die Ausführungsform ist aber auch auf nicht vorgeformte aufnehmende Elemente 101 anwendbar. In Fig. 2a ist das energieübertragende Element 11 fast in das aufnehmende Element 101 eingeführt. Fig. 2b zeigt, wie das aufnehmende Element 101 das energieübertragende Element 11 nach dem Einführen umhüllt und/oder an das energieübertragende Element 11 nach dem Einführen anliegt. Dabei stehen beide Elemente 101, 11 nach dem Einführen in direktem (und im Wesentlichen oder gänzlich spaltfreiem) Kontakt zueinander. Es handelt sich somit um eine direkte Umhüllung durch das aufnehmende Element 101 und/oder in ein direktes Anliegen des aufnehmenden Elements 101 an das energieübertragende Element 11. In Fig. 2b nimmt das aufnehmende Element 101 das energieübertragende Element 11 vollständig auf. Ähnlich kann auch nur ein Teil des gesamten energieübertragenden Elements 11 aufgenommen werden (teilweises Aufnehmen).

Zum Einführen wie oben beispielhaft dargestellt ist das aufnehmende Element 101 ausgestaltet, das energieübertragende Element 11 durch eine der folgenden Schritte zumindest teilweise aufzunehmen: durch Anwenden eines äußeren Anpressdrucks auf das energieübertragende Element 11; durch Anwenden eines Überdrucks in einem Lyse-Behälter mit der dem Lysieren zu unterziehenden Probe; durch ein kombiniertes Anwenden eines Überdrucks um einen Lyse-Behälter mit der dem Lysieren zu unterziehenden Probe und einer Gegenkraft auf das energieübertragende Element 11 von außen; oder durch Anwenden eines Unterdrucks um das energieübertragende Element 11. Somit wird ein flexibles Ausgestalten des Aufnehmens des energieübertragenden Elements 11 in das aufnehmende Element 101 ermöglicht. Die Wahl der jeweiligen Methode zum Aufnehmen kann in Abhängigkeit der Umgebung und der weiteren Ausgestaltung der Vorrichtung 10 bestimmt werden.

Bei dem zumindest teilweisen Aufnehmen des energieübertragenden Elements 11 kann sich das aufnehmende Element 101 in die Richtung des energieübertragenden Elements 11 wölben und dabei mit dem aufgenommenen Teil des energieübertragenden Elements 11 einen direkten und/oder (im Wesentlichen) spaltfreien Kontakt aufbauen.

Im Nachfolgenden werden weitere Ausgestaltungsformen der Vorrichtung 10 erläutert. Hierzu ist anzumerken, dass die oben beschriebenen Eigenschaften und positiven Wirkungen auch auf die nachfolgenden Ausgestaltungsformen zutreffen, es sei denn, dass bezüglich einer der nachfolgenden Ausgestaltungsformen ausdrücklich Anderweitiges oder Konkreteres erläutert wird. Ferner ist anzumerken, dass die bezüglich der nachfolgenden Ausgestaltungsformen erläuterten Eigenschaften und Merkmale die Eigenschaften und Merkmale der oben erläuterten Vorrichtung 10 ergänzen.

Wie den nachfolgenden Ausführungsformen zu entnehmen ist, kann die Vorrichtung 10 beispielsweise eines der Folgenden sein: ein Deckel für einen Lyse-Behälter; ein Adapter für einen Lyse-Behälter; ein Lyse-Behälter; oder ein Chiplabor (englisch: "Lab-on-a-Chip"). Gemäß einer Ausführungsform der vorliegenden Erfindung weist das Chiplabor 10 eine integrierte Kartusche zum Prozessieren von Analysen auf, die das aufnehmende Element 101 aufweisen kann. Gemäß einer weiteren Ausführungsform, kann die Vorrichtung 10 die oben erwähne Kartusche zum Prozessieren von Analysen sein. Durch diese Ausführungsformen wird ein flexibles und den Gegebenheiten und Bedürfnissen angepasstes Umsetzen der vorliegenden Erfindung ermöglicht.

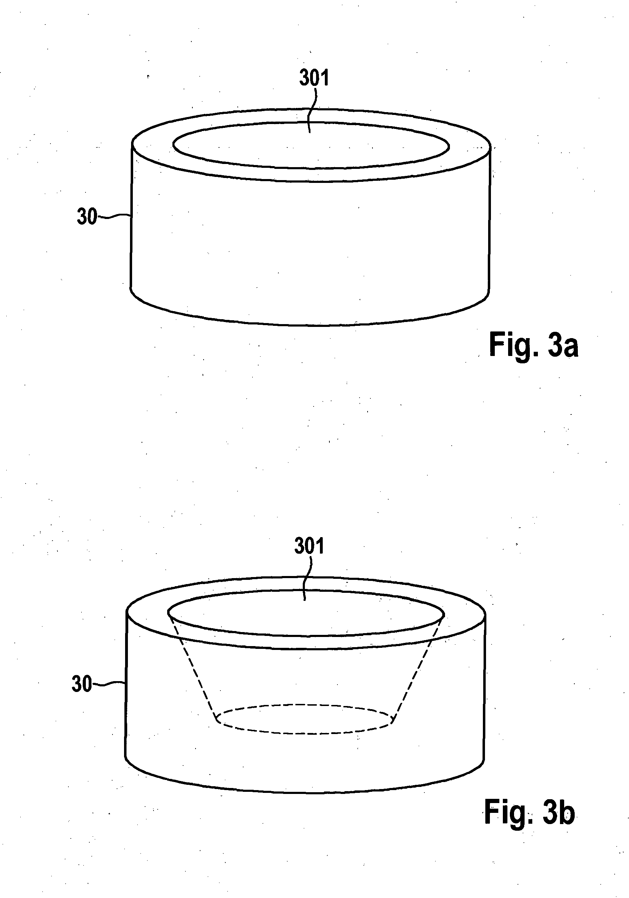

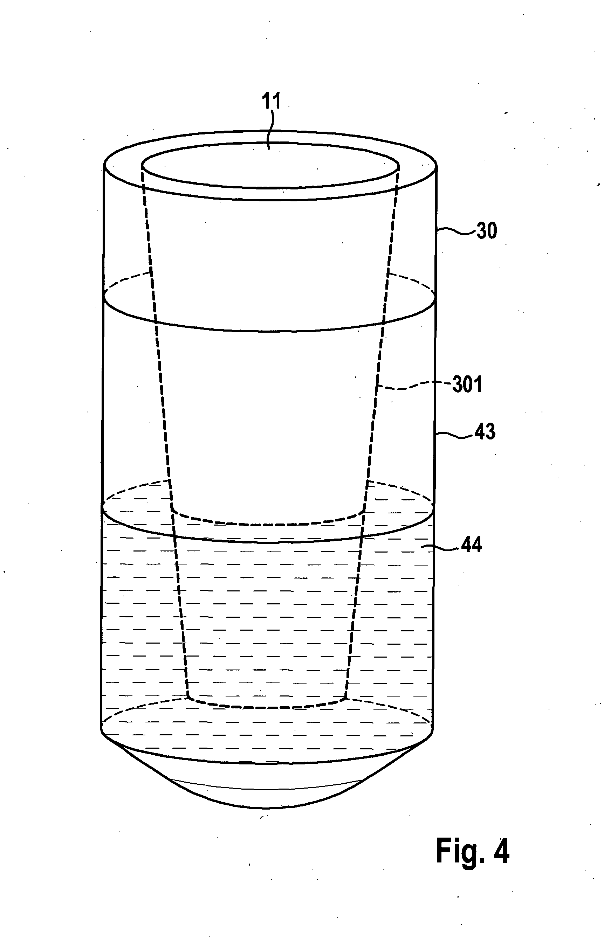

Fig. 3 zeigt eine Vorrichtung 30 gemäß einer Ausführungsform der vorliegenden Erfindung, wobei die Vorrichtung 30 zum Verwenden beim Lysieren mittels Ultraschalls ausgestaltet ist und ein Deckel oder ein Adapter für einen Lyse-Behälter ist. Fig. 4 zeigt die Vorrichtung 30, wenn das aufnehmende Element 301 der Vorrichtung 30 das energieübertragende Element 11 zumindest teilweise zum Lysieren einer Probe 44 aufgenommen hat. Die Probe 44 befindet sich dabei in einem Lyse-Behälter 43, der mit dem Deckel oder Adapter 30 verbunden ist. Wenn das aufnehmende Element 301 das energieübertragende Element 11 (zumindest teilweise) aufgenommen hat (siehe Fig. 4), ragt das aufnehmende Element 301, welches das energieübertragende Element 11 (direkt) umhüllt und/oder welches an das energieübertragende Element (direkt) anliegt, in die dem Lysieren zu unterziehende Probe 44 hinein. Die Probe 44 kann durch das umhüllte energieübertragende Element 11 dem Lysieren unterzogen werden. Wie bereits erwähnt, kann das Lysieren beispielsweise mittels Ultraschallübertragung durch das energieübertragende Element 11 erfolgen.

Gemäß dieser Ausführungsform wird das energieübertragende Element 11 durch den Deckel oder Adapter 30 von der Probeflüssigkeit 44, die das aufzuschließende oder zu lysierende Material enthält, getrennt. Der Deckel oder Adapter 30 weist das aufnehmende Element 301 auf, welches/welche bezüglich der Form des energieübertragenden Elements 11 vorgeformt (siehe Fig. 3b) sein kann oder im Wesentlichen flach oder eben (siehe Fig. 3a) ausgestaltet sein kann. Das aufnehmende Element 301 nimmt durch den Kontakt zu dem energieübertragenden Element 301 und durch die Verbindungskraft (z.B. Druck, Unterdruck, Klemmung, und/oder Überziehen) die erforderliche Form zum (zumindest teilweisen) Überziehen des energieübertragenden Elements 11 und/oder zum (zumindest teilweisen) Anliegen an das energieübertragende Element 11 ein. Das aufnehmende Element 301 kann aus Polymer oder Elastomer (z.B. Gummi) bestehen und kann beispielsweise eine Dicke von 10 µm bis 400 µm haben. Das aufnehmende Element 301 kann sich zumindest um den vorderen Teil des energieübertragenden Elements 11 (direkt) schließen und somit einen (direkten) Überzug für dieses/diese realisieren. Die vorübergehende Verbindung zwischen dem energieübertragenden Element 11 und der Außenseite des Deckels oder des Adapters 30 (bzw. dem aufnehmenden Element 301) kann über verschiedene Mechanismen vermittelt werden, z.B. durch: Anwenden eines äußeren Anpressdrucks auf das energieübertragende Element 11 (z.B. mittels Federn, Schrittmotor(en), Klemmung(en), Pressung(en)); Anwenden eines Überdrucks um den Lyse-Behälter 43 in Kombination mit dem Anwenden einer Gegenkraft auf das energieübertragende Element 11 von außen; oder Anwenden eines Unterdrucks um das energieübertragende Element 11.

Ferner kann der Deckel oder der Adapter 30 so ausgestaltet sein, dass der Deckel oder der Adapter 30 eine vorübergehende oder (fest) dichtende Funktion zwischen der Probeflüssigkeit 44 und der Außenwelt (d.h. äußere Umgebung des Lyse-Behälters 43 und des Deckels oder Adapters 30) ausübt. Auch dieses kann über verschiedene Mechanismen realisiert werden, z.B.: das aufnehmende Element 301 selbst ist ausgestaltet, die dichtende Funktion auszuführen; und/oder das Element 301 ist ein starres Element des Deckels oder des Adapters 30, der beispielsweise durch Schrauben, Klemmen, und/oder Rasten mit dem Lyse-Behälter 43 verbunden werden kann.

Der Deckel oder der Adapter 30 können derart ausgebildet sein, dass sie auf handelsübliche Lyse-Behälter 43 aufgesteckt und/oder verschraubt werden können oder während der Aktuation an handelsübliche Lyse-Behälter 43 angedrückt werden können. Wie bereits erläutert, kann der Deckel oder der Adapter 30 eine Dichtfunktion bereitstellen. Der Deckel oder der Adapter 30 können als Einwegartikel ausgestaltet sein.

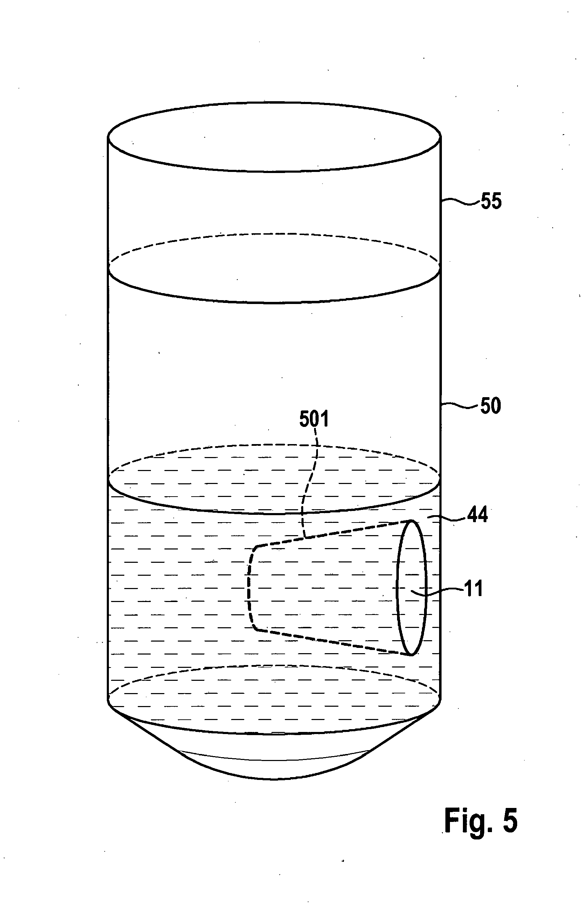

Fig. 5 zeigt eine zum Lysieren (einer dem Lysierenden zu unterzeihenden Probe) ausgestaltete Vorrichtung 50 gemäß einer Ausführungsform der vorliegenden Erfindung, wobei die Vorrichtung 50 ein Lyse-Behälter ist. Das Lysieren kann beispielsweise mittels Ultraschalls erfolgen. Fig. 5 zeigt eine Situation, in der das energieübertragende Element 11 in das aufnehmende Element 501 des Lyse-Behälters 50 eingeführt ist. Es ist anzumerken, dass die Eigenschaften, Merkmale und Wirkungen, die vorstehend oder nachfolgend bezüglich weiterer Ausgestaltungsformen erläutert sind und die nicht explizit gegensätzlich oder anders bezüglich der Eigenschaften, Merkmale und Wirkungen der vorliegenden Ausführungsform sind, die vorliegende Ausgestaltungsform ergänzen, auch wenn nicht ausdrücklich erwähnt, um die vorliegende Beschreibung kurz zu halten.

Gemäß der vorliegenden Ausführungsform ist das aufnehmende Element 501 im Lyse-Behälter 50 (fest) eingefügt und wird durch Unterdruck um das energieübertragende Element 11 und/oder durch Überdruck im Lyse-Behälter 50 in die zu lysierende Probe 44 (luftblasenfrei) eingetaucht. Das aufnehmende Element 501 stellt somit eine Schnittstelle zu dem/der extern angelegten energieübertragenden Element 11 dar. Die Verbindung zwischen dem aufnehmenden Element 501 und dem energieübertragenden Element 11 wird wie oben bezüglich der vorstehenden Ausführungsformen dargestellt realisiert.

Das aufnehmende Element 501 kann beispielsweise durch eine der folgenden Methoden in den Lyse-Behälter 50 integriert werden: Laserdurchstrahlschweißen, Laminieren, Ultraschallschweißen, Kleben und/oder Folientiefziehen.

Der Lyse-Behälter 50 kann mit einem Deckel 55 verbunden sein, um Einflüsse von außen auf die Probeflüssigkeit 44 zu vermeiden. Ferner kann der Lyse-Behälter 50 auch als Einwegbehälter ausgestaltet werden.

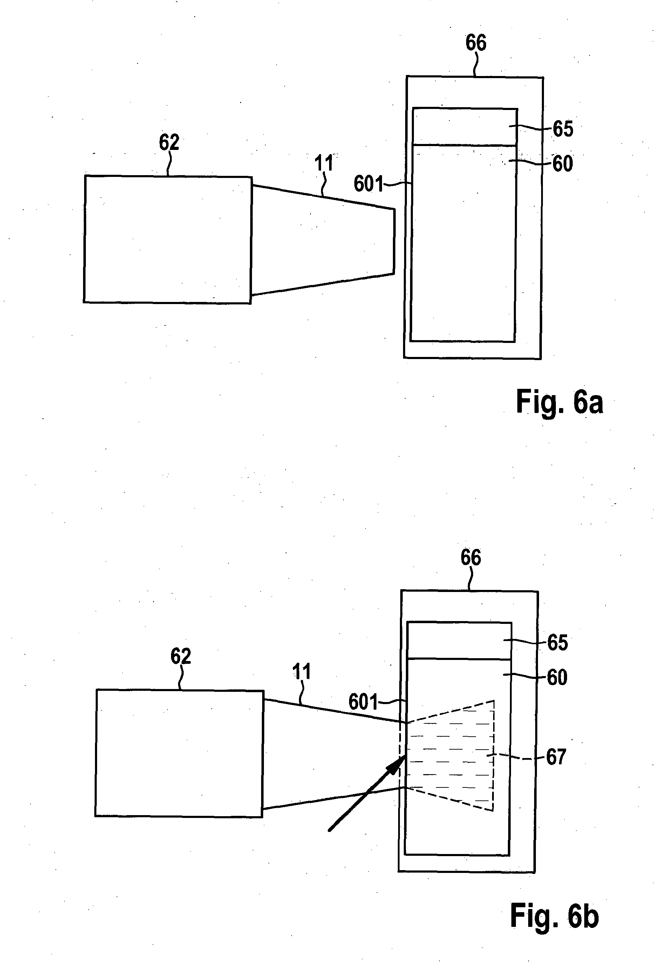

Fig. 6 zeigt eine zum Lysieren (einer dem Lysieren zu unterziehenden Probe) ausgestaltete Vorrichtung 60 gemäß einer Ausführungsform der vorliegenden Erfindung, wobei die Vorrichtung 60 ein Lyse-Behälter ist und wobei das Lysieren mittels Ultraschall vorgenommen werden kann. Es ist anzumerken, dass die Eigenschaften, Merkmale und Wirkungen, die vorstehend oder nachfolgend bezüglich weiterer Ausgestaltungsformen erläutert sind und die nicht explizit gegensätzlich oder anders bezüglich der Eigenschaften, Merkmale und Wirkungen der vorliegende Ausführungsform sind, die vorliegende Ausgestaltungsform ergänzen, auch wenn nicht ausdrücklich erwähnt, um die vorliegende Beschreibung kurz zu halten.

Der Lyse-Behälter 60 ist in einer Lyse-Kammer 66 angeordnet. Die dem energiegebenden Element 11 zugewandte Seite/Wand des Lyse-Behälters ist (zumindest zum Teil) als ein aufnehmendes Element 601 ausgestaltet. Das aufnehmende Element 601 kann eine Polymermembran sein. Die Polymermembran 601 kann beispielsweise aus PC, COP, COC, PP, PE, PMMA, PET, PEN, Elastomer (z.B. Gummi, Silicon, TPE) bestehen. Ferner kann die Polymermembran 601 eine Dicke von 10 µm bis 1 mm haben. Wenn die Polymembran 601 beispielsweise aus PC, COP oder COC besteht, kann sie beispielsweise eine Dicke 300 µm bis 500 µm haben. Im Vergleich zu anderen Ausführungsformen können gemäß der vorliegenden Ausführungsform steifere Membranen verwendet werden, die eine geringere Dämpfung aufweisen.

An das aufnehmende Element 601 wird das energieübertragende Element 11 angelegt oder das energieübertragende Element 11 wird in die Nähe des aufnehmenden Elements 601 gebracht (siehe Fig. 6a). Der Spalt zwischen den beiden Elementen 11, 601 kann beispielsweise ca. 0,1 mm breit sein. Daraufhin wird der Lyse-Behälter 60 mit Druck (z.B. Luft oder flüssiger Inhalt) beaufschlagt. Der Absolutdruck kann dabei beispielsweise 0,5 bar bis 2 bar sein. Dabei entsteht im Lyse-Behälter 60 Überdruck, wodurch sich das aufnehmende Element 601 in die Richtung des energiegebenden Elements 11 wölbt und mit diesem einen (im Wesentlichen) spaltfreien Kontakt aufbaut (siehe Fig. 6b). Der (im Wesentlichen) spaltfreie Kontakt ist in Fig. 6b mit einem Pfeil zwischen den beiden Elementen 11 und 601 markiert. Das Beaufschlagen des Drucks wird mit dem dickeren, auf die Lyse-Kammer 66, bzw. auf den Lyse-Behälter 60 gerichteten Pfeil verdeutlicht.

Das energieübertragende Element 11 kann beispielsweise als Resonator, Sonotrode, Horn, Lambda-halbe oder Lambda-Wandler aufgebaut sein.

Daraufhin wird der Ultraschallgenerator (nicht gezeigt) bis zu mehrere Minuten betätigt, um die im Lyse-Behälter 60 befindliche Probeflüssigkeit zu lysieren. Der Ultraschallgenerator kann beispielsweise mit 30 kHz betätigt werden, wobei niedrigere oder höhere Frequenzen von 20 kHz bis zu 1 MHz ebenfalls möglich sind. Bei 40 kHz wurde mehr DNA-Fragmentierung beobachtet, bei 20 kHz tritt eine starke Geräuschbelastung auf. Dabei kann der Energieeintrag kontinuierlich oder gepulst erfolgen. Der Ultraschallgenerator ist mit dem Ultraschallwandler 62 gekoppelt, welcher wiederum Schall an das energiegebende Element 11 überträgt. Durch das Beschallen der Probeflüssigkeit in dem Lyse-Behälter 60 werden Scherkräfte und/oder Kavitation 67 erzeugt, durch die das Lysieren der Probeflüssigkeit in dem Lyse-Behälter 60 bewirkt wird.

Anschließend kann die dem Lysieren unterzogene Probeflüssigkeit aus der Lysekammer 66 entnommen werden und in einem Chiplabor (englisch: Lab-on-a-Chip) weiterbearbeitet werden. Dazu sind ggf. eine Einlass- (Zuführung ggf. zusätzliches Lysemedium) und eine Auslassöffnung (z.B. für die Weiterbehandlung) erforderlich. In Fig. 6 ist diese mit einem Deckel 65 beispielhaft verdeckt und verschlossen.

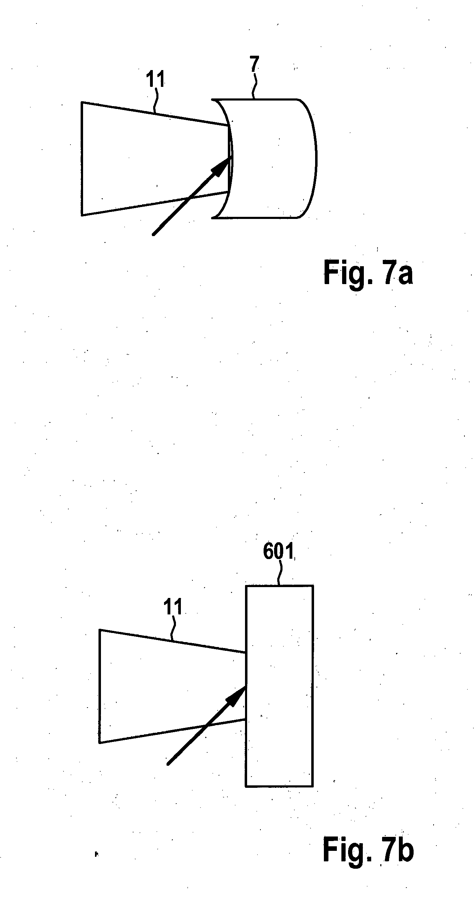

Mit der vorliegenden Ausführungsform können auch flachere Lyse-Behälter 60 verwendet werden, die bei anderen Lysierverfahren den Nachteil von schwer reproduzierbaren Ergebnissen mit sich ziehen, da sich flache Behälterwände bei Andruck eines energieübertragenden Elements 11 so durchbiegen, dass in der Mitte des energieübertragenden Elements 11 der Kontakt zwischen der Behälterwand und dem energieübertragenden Element 11 abreißt; siehe Fig. 7a, in der ein Spalt (mit einem Pfeil markiert) bei der Kontaktbildung zwischen dem energieübertragenden Element 11 und der Behälterwand 7 entsteht. Fig. 7b zeigt das spaltlose Bilden eines Kontakts gemäß der vorliegenden Ausführungsform. Bei der Verwendung weniger elastischer aufnehmenden Elemente 601 (z.B. Polymer-Membranen) wird die Kontaktbildung wird umso schlechter, je höher der Anpressdruck des energieübertragenden Elements 11 zu der Wand eines Lyse-Behälters gewählt wird. Die dann resultierenden ringförmigen Kontaktflächen können bei Ultraschallbeaufschlagung leicht überhitzen und die Ankopplung wird unreproduzierbar. Dieses Phänomen wird umso kritischer, je dünner die Wandung des Lyse-Behälters bzw. je dünner das aufnehmende Element 601 ist und je weniger flexibel (z.B.je dicker) das aufnehmende Element 601 (z.B. Membran) ist.

Mit der vorliegenden Ausführungsform wird dieses Ankopplungsproblem gelöst, indem ein flaches energieübertragendes Element in der Nähe des aufnehmenden Elements 601 des Lyse-Behälters 60 angebracht wird und der Lyse-Behälter 60 so mit Druck (z.B. Luft oder flüssigem Inhalt) beaufschlagt wird, dass sich das aufnehmende Element 601 an das energieübertragende Element 11 anlegt und den Spalt zwischen dem energieübertragenden Element 11 und dem Lyse-Behälter 60 zuverlässig schließt. Der so aufgebaute Kraftschluss zwischen den beiden Elementen 11, 601 führt zu einer zuverlässigen Energie-(z.B. Ultraschall-) Einkopplung. Ferner lassen sich auf diese Weise auch Keilfehler zwischen dem energieübertragenden Element 11 und dem Lyse-Behälter 60 bzw. dem aufnehmenden Element 601 (z.B. aufgrund Halterungstoleranzen oder Kartuschen-Fertigungstoleranzen) ausgleichen. Für einen optimalen Kraftschluss kann darüber hinaus auch die Geometrie der Spitze des energieübertragenden Elements, die auf das aufnehmende Element bei ihrer Aufnahme trifft), auf die Geometrie des aufnehmenden Elements unter Belastung ausgelegt werden.

Der Lyse-Behälter 60 kann auch als Einwegbehälter bereitgestellt werden.

Zusammenfassend sind die positiven Wirkungen der vorliegenden Ausführungsform zumindest wie folgt. Gemäß der vorliegenden Ausführungsform können auch flache aufnehmende Elemente 601 verwendet werden. Das Ankoppeln des aufnehmenden Elements 601 an das energieübertragende Element 11 kann auf eine einfache Weise vollzogen werden. Die Ausführungsform erlaubt mit großer Sicherheit das Entstehen von Kavitation und/oder Scherkräften zwecks Lysierung. Es lassen sich sehr dünne aufnehmende Elemente 601 (z.B. Membranen) einsetzen, welche beispielsweise die Dicke von 10 µm bis 1mm und dabei beispielsweise eine Dicke von 200 µm bis 500 µm, 50 µm bis 1 mm haben können. Flache aufnehmende Elemente 601 (z.B. Membranen) lassen sich vollflächig über die Oberfläche des energieübertragenden Elements 11 kühlen und sind haltbarer als nur punktuell oder ringförmig anliegende aufnehmende Elemente 601 (z.B. Membranen). Das Lysieren ist schnell und materialsparend auszuführen.

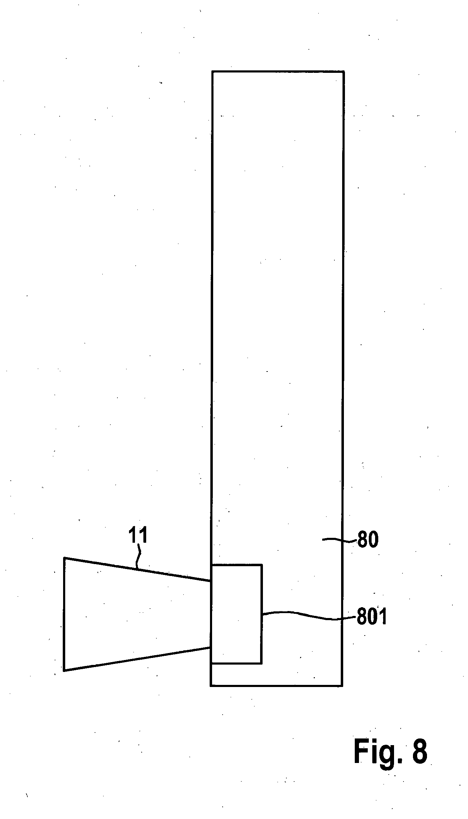

Fig. 8 zeigt eine zum Lyiseren (einer dem Lysieren zu unterziehenden Probe) ausgestaltete Vorrichtung 80 gemäß einer Ausführungsform der vorliegenden Erfindung, wobei die Vorrichtung 80 ein Chiplabor (englisch: "Lab-on-Chip-System") ist. Das Lysieren kann beispielsweise mittels Ultraschall vorgenommen werden. Es ist anzumerken, dass die Eigenschaften, Merkmale und Wirkungen, die vorstehend oder nachfolgend bezüglich weiterer Ausgestaltungsformen erläutert sind und die nicht explizit gegensätzlich oder anders bezüglich der Eigenschaften, Merkmale und Wirkungen der vorliegende Ausführungsform sind, die vorliegende Ausgestaltungsform ergänzen, auch wenn nicht ausdrücklich erwähnt, um die vorliegende Beschreibung kurz zu halten. Das Chiplabor 80 weist ein aufnehmendes Element 801 auf, das wie vorstehend ausgeführt elastisches und deformierbares Element und/oder eine Membran sein kann. Das aufnehmende Element 801 stellt eine Schnittstelle des Chiplabors 80 zu einem energieübertragenden Element 11 dar. Im Wesentlichen weist das aufnehmende Element 801 die gleichen Eigenschaften, Merkmale und Wirkungen wie die oben erläuterten aufnehmenden Elemente 101, 301, 501, 601 auf.

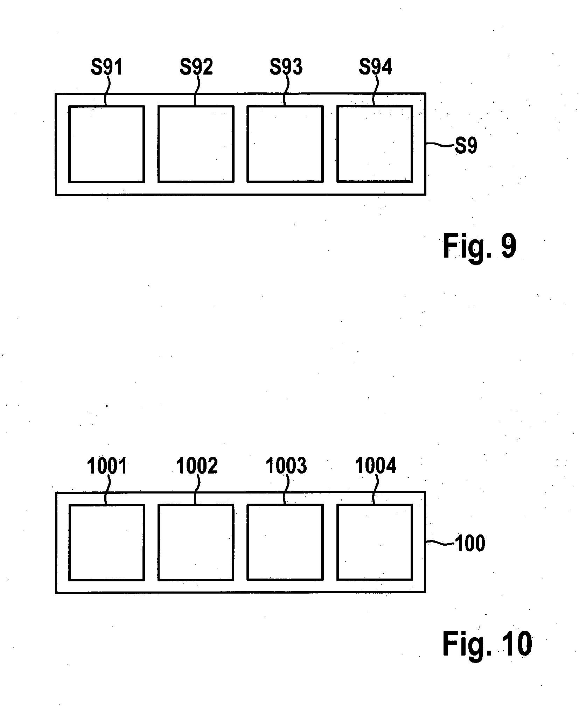

Fig. 9 zeigt gemäß einer Ausführungsform der vorliegenden Erfindung die Schritte eines Verfahrens zum Einführen eines energieübertragenden Elements 11 in ein aufnehmendes Element 101, 301, 501, 601, 801. Das Verfahren weist in erster Linie den Schritt S9 des zumindest teilweisen Einführens eines energieübertragenden Elements 11 in ein aufnehmendes Element 101, 301, 501, 601, 801 auf, wobei durch das zumindest teilweise Einführen das aufnehmende Element 101, 301, 501, 601, 801 das energieübertragende Element 11 derart zumindest teilweise aufnimmt, sich das aufnehmende Element 101, 301, 501, 601, 801 um den aufgenommenen Teil des energieübertragenden Elements 11 legt und den aufgenommenen Teil des energieübertragenden Elements 11 umhüllt und/oder dass sich das aufnehmende Element 101, 301, 501, 601, 801 an den aufgenommenen Teil des energieübertragenden Elements 11 legt, wobei durch das zumindest teilweise Einführen das aufnehmende Element 101, 301, 501, 601, 801 mit dem aufgenommenen energieübertragenden Element 11 zum Lysieren einer dem Lysieren zu unterziehenden Probe bereitgestellt werden. Das Lysieren kann z.B. mittels Ultraschalls erfolgen. Mit diesem Schritt S9 wird das Lysieren mittels Ultraschalls ausgelöst, wenn das energieübertragende Element 11, welches in das aufnehmende Element 101, 301, 501, 601, 801 eingeführt ist, in eine zu lysierende Probe eingetaucht wird oder mit der zu lysierenden Probe in Kontakt steht.

Das zumindest teilweise Einführen S9 wird derart ausgeführt, dass das aufnehmende Element 101, 301, 501, 601, 801 sich direkt um und/oder an den aufgenommenen Teil des energieübertragenden Elements 11 legt und in einem direkten ((im Wesentlichen) spaltfreien) Kontakt zu dem aufgenommenen Teil des energieübertragenden Elements 11 ist.

Gemäß dem Verfahren der vorliegenden Ausführungsform kann das zumindest teilweise Einführen S9 durch einen der Folgenden ausgeführt werden: durch Anwenden eines äußeren Anpressdrucks auf das energieübertragende Element 11 in Schritt S91; durch Anwenden eines Überdrucks in einem Lyse-Behälter 43, 50, 60 mit einer dem Lysieren zu unterziehenden Probe 44 in Schritt S92; durch ein kombiniertes Anwenden eines Überdrucks um einen Lyse-Behälter 43, 50, 60 mit einer dem Lysieren zu unterziehenden Probe 44 und einer Gegenkraft auf das energieübertragende Element 11 von außen in Schritt S93; oder durch Anwenden eines Unterdrucks um das energieübertragende Element 11 in Schritt S94.

In Schritt S9 kann das zumindest teilweise Einführen derart ausgeführt werden, dass sich das aufnehmende Element 101, 301, 501, 601, 801 bei dem zumindest teilweisen Aufnehmen des energieübertragenden Elements 11 in die Richtung des energieübertragenden Elements 11 wölbt und mit dem aufgenommenen Teil des energieübertragenden Elements 11 einen (im Wesentlichen) spaltfreien Kontakt aufbaut.

Weitere Details zu den Schritten des Verfahrens der Fig. 9 können den obigen Ausführungsformen entnommen werden.

Fig. 10 zeigt gemäß einer Ausführungsform eine Vorrichtung 100 . Im Wesentlichen ist die Vorrichtung 100 ausgestaltet, das zumindest teilweise Einführen des energieübertragenden Elements 11 in das aufnehmende Element 101, 301, 501, 601, 801 auszuführen. Dabei kann die Vorrichtung 100 die folgenden Module/Elemente aufweisen: das Modul/Element 1001 ; das Modul/Element 1002 ; das Modul/Element 1003 ; und/oder das Modul/Element 1004.

Im Hinblick auf die vorstehend erläuterten Figuren wird ferner deutlich, dass die vorliegende Erfindung auch ein System umfasst, das die Vorrichtung 10, 30, 50, 60, 80, die zum Verwenden beim Lysieren einer dem Lysieren zu unterziehenden Probe ausgestaltet ist, und das das energieübertragende Element 11 aufweist.

Die vorstehend erläuterten Ausführungsformen mit den dort erläuterten spezifischen Aspekten sind mit einander kombinierbar. Insbesondere sind die Eigenschaften, Funktionen und/oder Ausgestaltungen der aufnehmenden Elemente 101, 301, 501, 601, 801 gleich oder zumindest ähnlich. Die vorliegende Erfindung ermöglicht ein kontaminationsfreies und reproduzierbares Lysieren. Ferner kann das Lysieren auf eine schnelle und effiziente Weise vorgenommen werden. Zusätzlich wird durch die vorliegende Erfindung ein sicheres Einkoppeln der Energie (z.B. Ultraschalls) in die zu lysierende Probe gewährleistet.

## Patentansprüche

1. Vorrichtung (10, 30, 50, 60, 80), die zum Verwenden beim Lysieren einer dem Lysieren zu unterziehenden Probe (44) ausgestaltet ist, wobei die Vorrichtung (10, 30, 50, 60, 80) ein aufnehmendes Element (101, 301, 501, 601, 801) aufweist, das als Membran ausgestaltet ist und/oder vollständig oder zumindest teilweise elastisch und deformierbar ist, wobei das aufnehmende Element (101, 301, 501, 601, 801) ausgestaltet ist:
- ein energieübertragendes Element (11) zumindest teilweise derart aufzunehmen, dass sich das aufnehmende Element (101, 301, 501, 601, 801) um den aufgenommenen Teil des energieübertragenden Elements (11) legt und den aufgenommenen Teil des energieübertragenden Elements (11) umhüllt und/oder dass sich das aufnehmende Element (101, 301, 501, 601, 801) an den aufgenommenen Teil des energieübertragenden Elements (11) legt; und
- mit dem energieübertragenden Element (11), dessen aufgenommene Teil durch das aufnehmende Element (101, 301, 501, 601, 801) umhüllt ist und/oder an dessen aufgenommenen Teil das aufnehmende Element (101, 301, 501, 601, 801) anliegt, das Lysieren zu bewirken, wenn das aufnehmende Element (101, 301, 501, 601, 801), das den aufgenommenen Teil des energieübertragenden Elements (11) umhüllt und/oder das an den den aufgenommenen Teil des energieübertragenden Elements (11) anliegt, in die dem Lysieren zu unterziehende Probe (44) eingetaucht ist oder mit der dem Lysieren zu unterziehenden Probe (44) in Kontakt steht,
wobei das aufnehmende Element (101, 301, 501, 601, 801) ausgestaltet ist, das energieübertragende Element (11) durch einen der Folgenden zumindest teilweise aufzunehmen:
- durch ein Anwenden eines äußeren Anpressdrucks auf das energieübertragende Element (11);
- durch ein Anwenden eines Überdrucks in einem Lyse-Behälter (43, 50, 60) mit der dem Lysieren zu unterziehenden Probe (44);
- durch ein kombiniertes Anwenden eines Überdrucks um einen Lyse-Behälter (43, 50, 60) mit der dem Lysieren zu unterziehenden Probe (44) und einer Gegenkraft auf das energieübertragende Element (11) von außen; oder
- durch ein Anwenden eines Unterdrucks um das energieübertragende Element (11);
und/oder wobei bei dem zumindest teilweisen Aufnehmen sich das aufnehmende Element (101, 301, 501, 601, 801) in die Richtung des energieübertragenden Elements (11) wölbt und mit dem aufgenommenen Teil des energieübertragenden Elements (11) einen spaltfreien oder im Wesentlichen spaltfreien Kontakt aufbaut.

2. Vorrichtung (10, 30, 50, 60, 80) nach Anspruch 1, wobei die Vorrichtung (10, 30, 50, 60, 80) eines der Folgenden ist: ein Deckel (30) für einen Lyse-Behälter (43); ein Adapter (30) für einen Lyse-Behälter (43); ein Lyse-Behälter (50, 60); oder ein Chiplabor (80).

3. Vorrichtung (10, 30, 50, 60, 80) nach Anspruch 1 oder 2, wobei das aufnehmende Element (101, 301, 501, 601, 801) ausgestaltet ist, sich direkt um und/oder an den aufgenommenen Teil des energieübertragenden Elements (11) zu legen und in direktem Kontakt zu dem aufgenommenen Teil des energieübertragenden Elements (11) zu sein.

4. Vorrichtung (10,30, 50, 60, 80) nach einem der vorstehenden Ansprüche, wobei das energieübertragende Element (11) eine Sonotrode oder ein Horn ist.

5. Vorrichtung (10, 30, 50, 60, 80) nach einem der vorstehenden Ansprüche, wobei:
- das aufnehmende Element (101, 301, 501, 601, 801) bezüglich des aufzunehmenden energieübertragenden Elements (11) vorgeformt ist;
- das aufnehmende Element (101, 301, 501, 601, 801) aus einem Polymer oder aus Elastomer besteht; und/oder
- das aufnehmende Element (101, 301, 501, 601, 801) eine Dicke von 10 µm bis 1mm aufweist, wobei die Dicke ferner 10 µm bis 400 µm, 50 µm bis 1 mm oder 300 µm bis 400 µm sein kann.

6. Verfahren, das ein zumindest teilweises Einführen (S9) eines energieübertragenden Elements in ein aufnehmendes Element aufweist, wobei das aufnehmende Element als Membran ausgestaltet ist und/oder vollständig oder zumindest teilweise elastisch und deformierbar ist, wobei durch das zumindest teilweise Einführen (S9) das aufnehmende Element das energieübertragende Element derart zumindest teilweise aufnimmt, dass sich das aufnehmende Element um den aufgenommenen Teil des energieübertragenden Elements legt und den aufgenommenen Teil des energieübertragenden Elements umhüllt und/oder dass sich das aufnehmende Element an den aufgenommenen Teil des energieübertragenden Elements legt, wobei durch das zumindest teilweise Einführen (S9) das aufnehmende Element mit dem aufgenommenen energieübertragenden Element zum Lysieren einer dem Lysieren zu unterziehenden Probe bereitgestellt werden, wobei das zumindest teilweise Einführen (S9) durch einen der Folgenden ausgeführt wird:
- durch Anwenden (S91) eines äußeren Anpressdrucks auf das energieübertragende Element;
- durch Anwenden (S92) eines Überdrucks in einem Lyse-Behälter mit einer dem Lysieren zu unterziehenden Probe;
- durch ein kombiniertes Anwenden (S93) eines Überdrucks um einen Lyse-Behälter mit einer dem Lysieren zu unterziehenden Probe und einer Gegenkraft auf das energieübertragende Element von außen; oder
- durch Anwenden (S94) eines Unterdrucks um das energieübertragende Element;
und/oder wobei das Einführen (S9) derart ausgeführt wird, dass der aufgenommene Teil das aufnehmende Element sich bei dem zumindest teilweisen Aufnehmen des energieübertragenden Elements in die Richtung des energieübertragenden Elements wölbt und mit dem aufgenommenen Teil des energieübertragenden Elements einen spaltfreien oder im Wesentlichen spaltfreien Kontakt aufbaut.

7. Verfahren nach Anspruch 6, wobei das zumindest teilweise Einführen (S9) derart ausgeführt wird, dass das aufnehmende Element sich direkt um und/oder an den aufgenommenen Teil des energieübertragenden Elements legt und in einem direkten Kontakt zu dem aufgenommenen Teil des energieübertragenden Elements steht.

8. System, das eine Vorrichtung (10, 30, 50, 60, 80) nach einem der Ansprüche 1 bis 5 und das das energieübertragende Element (11) aufweist.

## Claims

1. Device (10, 30, 50, 60, 80), which is embodied to be used for lysing a sample (44) to be subjected to lysis, the device (10, 30, 50, 60, 80) comprising a holding element (101, 301, 501, 601, 801), which is embodied as a membrane and/or is completely or at least partly elastic and deformable, the holding element (101, 301, 501, 601, 801) being embodied as:
- to hold an energy-transmitting element (11) at least in part such that the holding element (101, 301, 501, 601, 801) lies around the held part of the energy-transmitting element (11) and envelops the held part of the energy-transmitting element (11) and/or that the holding element (101, 301, 501, 601, 801) lies against the held part of the energy-transmitting element (11); and
- to cause lysis with the energy-transmitting element (11), the held part of which is enveloped by the holding element (101, 301, 501, 601, 801) and/or against the held part of which the holding element (101, 301, 501, 601, 801) lies, when the holding element (101, 301, 501, 601, 801), which envelops the held part of the energy-transmitting element (11) and/or which lies against the held part of the energy-transmitting element (11), is immersed into the sample (44) to be subjected to lysis or is in contact with the sample (44) to be subjected to lysis,
wherein the holding element (101, 301, 501, 601, 801) is designed to at least partly hold the energy-transmitting element (11) by means of one of the following options:
- by applying external contact pressure on the energy-transmitting element (11);
- by applying positive pressure in a lysis container (43, 50, 60) with the sample (44) to be subjected to lysis;
- by combined application of positive pressure around a lysis container (43, 50, 60) with the sample (44) to be subjected to lysis and a counteracting force, applied from the outside, on the energytransmitting element (11); or
- by applying negative pressure around the energy-transmitting element (11);
and/or wherein, during the at least partial holding, the holding element (101, 301, 501, 601, 801) arches in the direction of the energy-transmitting element (11) and establishes a gap-free or substantially gap-free contact with the held part of the energy-transmitting element (11).

2. Device (10, 30, 50, 60, 80) according to Claim 1, wherein the device (10, 30, 50, 60, 80) is one of the following: a cover (30) for a lysis container (43); an adapter (30) for a lysis container (43); a lysis container (50, 60); or a lab on a chip (80).

3. Device (10, 30, 50, 60, 80) according to Claim 1 or 2, wherein the holding element (101, 301, 501, 601, 801) is embodied to lie directly around and/or against the held part of the energy-transmitting element (11) and to be in direct contact with the held part of the energy-transmitting element (11).

4. Device (10, 30, 50, 60, 80) according to one of the preceding claims, wherein the energy-transmitting element (11) is a sonotrode or a horn.

5. Device (10, 30, 50, 60, 80) according to one of the preceding claims, wherein:
- the holding element (101, 301, 501, 601, 801) is preformed in respect of the energy-transmitting element (11) to be held;
- the holding element (101, 301, 501, 601, 801) consists of a polymer or of elastomer; and/or
- the holding element (101, 301, 501, 601, 801) has a thickness of between 10 µm and 1 mm, wherein the thickness can furthermore be 10 µm to 400 µm, 50 µm to 1 mm or 300 µm to 400 µm.

6. Method, comprising at least partial insertion (S9) of an energy-transmitting element into a holding element, wherein the holding element is embodied as a membrane and/or is completely or at least partly elastic and deformable, wherein, as a result of the at least partial insertion (S9), the holding element at least partly holds the energy-transmitting element in such a way that the holding element lies around the held part of the energy-transmitting element and envelops the held part of the energy-transmitting element and/or that the holding element lies against the held part of the energy-transmitting element, wherein, as result of the at least partial insertion (S9), the holding element with the held energy-transmitting element are provided for lysing a sample to be subjected to lysis, wherein the at least partial insertion (S9) is carried out by one of the following options:
- by applying (S91) external contact pressure on the energy-transmitting element;
- by applying (S92) positive pressure in a lysis container with a sample to be subjected to lysis;
- by combined application (S93) of positive pressure around a lysis container with a sample to be subjected to lysis and a counteracting force, applied from the outside, on the energy-transmitting element; or
- by applying (S94) negative pressure around the energy-transmitting element;
and/or wherein the insertion (S9) is carried out in such a way that the held part arches the holding element when at least partly holding the energy-transmitting element in the direction of the energy-transmitting element and establishes a gap-free or substantially gap-free contact with the held part of the energy-transmitting element.

7. Method according to Claim 6, wherein the at least partial insertion (S9) is carried out in such a way that the holding element lies directly around and/or against the held part of the energy-transmitting element and is in direct contact with the held part of the energy-transmitting element.

8. System, comprising a device (10, 30, 50, 60, 80) according to one of Claims 1 to 5 and comprising the energy-transmitting element (11).

## Revendications

1. Dispositif (10, 30, 50, 60, 80) qui est configuré pour l'utilisation lors de la lyse d'un échantillon (44) devant être soumis à une lyse, le dispositif (10, 30, 50, 60, 80) présentant un élément de réception (101, 301, 501, 601, 801) qui est configuré sous forme de membrane et/ou qui est complètement ou au moins partiellement élastique et déformable, l'élément de réception (101, 301, 501, 601, 801) étant configuré :
- pour recevoir au moins en partie un élément de transmission d'énergie (11) de telle sorte que l'élément de réception (101, 301, 501, 601, 801) se place autour de la partie reçue de l'élément de transmission d'énergie (11) et enveloppe la partie reçue de l'élément de transmission d'énergie (11) et/ou de telle sorte que l'élément de réception (101, 301, 501, 601, 801) se place contre la partie reçue de l'élément de transmission d'énergie (11) ; et
- pour provoquer la lyse avec l'élément de transmission d'énergie (11) dont la partie reçue est enveloppée par l'élément de réception (101, 301, 501, 601, 801) et/ou contre la partie reçue duquel s'applique l'élément de réception (101, 301, 501, 601, 801), lorsque l'élément de réception (101, 301, 501, 601, 801) qui enveloppe la partie reçue de l'élément de transmission d'énergie (11) et/ou qui s'applique contre la partie reçue de l'élément de transmission d'énergie (11) est plongé dans l'échantillon (44) devant être soumis à la lyse ou est en contact avec l'échantillon (44) devant être soumis à la lyse,
l'élément de réception (101, 301, 501, 601, 801) étant configuré de manière à recevoir au moins en partie l'élément de transmission d'énergie (11) par l'une des mesures suivantes :
- application d'une pression d'application extérieure sur l'élément de transmission d'énergie (11) ;
- application d'une surpression dans un récipient de lyse (43, 50, 60) avec l'échantillon (44) devant être soumis à la lyse ;
- application combinée d'une surpression autour d'un récipient de lyse (43, 50, 60) avec l'échantillon (44) devant être soumis à la lyse et d'une force conjuguée sur l'élément de transmission d'énergie (11) depuis l'extérieur ; ou
- application d'une dépression autour de l'élément de transmission d'énergie (11) ;
et/ou dans le cas de la réception au moins partielle, l'élément de réception (101, 301, 501, 601, 801) se courbant dans la direction de l'élément de transmission d'énergie (11) et formant avec la partie reçue de l'élément de transmission d'énergie (11) un contact sans interstice ou essentiellement sans interstice.

2. Dispositif (10, 30, 50, 60, 80) selon la revendication 1, dans lequel le dispositif (10, 30, 50, 60, 80) est l'un des éléments suivants :
un couvercle (30) pour un récipient de lyse (43) ; un adaptateur (30) pour un récipient de lyse (43) ; un récipient de lyse (50, 60) ; ou un microlaboratoire sur puce (80).

3. Dispositif (10, 30, 50, 60, 80) selon la revendication 1 ou 2, dans lequel l'élément de réception (101, 301, 501, 601, 801) est configuré pour s'appliquer directement autour de et/ou contre la partie reçue de l'élément de transmission d'énergie (11) et pour être en contact direct avec la partie reçue de l'élément de transmission d'énergie (11).

4. Dispositif (10, 30, 50, 60, 80) selon l'une quelconque des revendications précédentes, dans lequel l'élément de transmission d'énergie (11) est une sonotrode ou une corne.

5. Dispositif (10, 30, 50, 60, 80) selon l'une quelconque des revendications précédentes, dans lequel :
- l'élément de réception (101, 301, 501, 601, 801) est préformé par rapport à l'élément de transmission d'énergie à recevoir (11) ;
- l'élément de réception (101, 301, 501, 601, 801) se compose d'un polymère ou d'un élastomère ; et/ou
- l'élément de réception (101, 301, 501, 601, 801) présente une épaisseur de 10 µm à 1 mm, l'épaisseur pouvant en outre être comprise entre 10 µm et 400 µm, 50 µm et 1 mm ou 300 µm et 400 µm.

6. Procédé présentant une insertion au moins partielle (S9) d'un élément de transmission d'énergie dans un élément de réception, l'élément de réception étant configuré sous forme de membrane et/ou étant complètement ou au moins partiellement élastique et déformable, l'élément de réception, par l'insertion au moins partielle (S9), recevant l'élément de transmission d'énergie au moins en partie de telle sorte que l'élément de réception s'applique autour de la partie reçue de l'élément de transmission d'énergie et enveloppe la partie reçue de l'élément de transmission d'énergie et/ou de telle sorte que l'élément de réception se place sur la partie reçue de l'élément de transmission d'énergie, l'élément de réception avec l'élément de transmission d'énergie reçu, par l'insertion au moins partielle (S9), étant disponibles pour la lyse d'un échantillon devant être soumis à la lyse, l'insertion au moins partielle (S9) étant effectuée par l'une des mesures suivantes :
- application (S91) d'une pression d'application extérieure sur l'élément de transmission d'énergie ;
- application (S92) d'une surpression dans un récipient de lyse avec un échantillon devant être soumis à la lyse ;
- application combinée (S93) d'une surpression autour d'un récipient de lyse avec un échantillon devant être soumis à la lyse et d'une force conjuguée sur l'élément de transmission d'énergie depuis l'extérieur ; ou
- application (S94) d'une dépression autour de l'élément de transmission d'énergie ;
et/ou l'insertion (S9) étant réalisée de telle sorte que l'élément de réception se courbe lors de la réception au moins partielle de l'élément de transmission d'énergie dans la direction de l'élément de transmission d'énergie et constitue avec la partie reçue de l'élément de transmission d'énergie un contact sans interstice ou essentiellement sans interstice.

7. Procédé selon la revendication 6, dans lequel l'insertion au moins partielle (S9) est réalisée de telle sorte que l'élément de réception s'applique directement autour de et/ou contre la partie reçue de l'élément de transmission d'énergie et soit en contact direct avec la partie reçue de l'élément de transmission d'énergie.

8. Système présentant un dispositif (10, 30, 50, 60, 80) selon l'une quelconque des revendications 1 à 5 et l'élément de transmission d'énergie (11).
